(19) Europäisches Patentamt
European Patent Office
Office européen des brevets

(11) Veröffentlichungsnummer: **0 175 641**
**B1**

# (12) EUROPÄISCHE PATENTSCHRIFT

(45) Veröffentlichungstag der Patentschrift: **24.02.88**

(21) Anmeldenummer: **85810367.4**

(22) Anmeldetag: **09.08.85**

(51) Int. Cl.⁴: **C 07 C 51/56**, C 07 C 51/567, C 07 C 57/13

## (54) Verfahren zur Herstellung von Dimethylmaleinsäureanhydrid.

(30) Priorität: **15.08.84 CH 3910/84**

(43) Veröffentlichungstag der Anmeldung: **26.03.86 Patentblatt 86/13**

(45) Bekanntmachung des Hinweises auf die Patenterteilung: **24.02.88 Patentblatt 88/8**

(84) Benannte Vertragsstaaten: **BE CH DE FR GB IT LI NL**

(56) Entgegenhaltungen:
**EP-A-0 078 239**
**GB-A-870 681**

**HELVETICA CHIMICA ACTA, Band 61, Nr. 7, November 1978, Seiten 2751-2753; M.E. BAUMANN et al.: "Eine decarboxylative Dimerisierung von Maleinsäureanhydrid zu Dimetzylmaleinsäureanhydrid"**

(73) Patentinhaber: **CIBA- GEIGY AG, Klybeckstrasse 141, CH- 4002 Basel (CH)**

(72) Erfinder: **Baumann, Marcus, Dr., Hammerstrasse 82, CH- 4057 Basel (CH)**
Erfinder: **Breitenstein, Werner, Dr., St. Galler-Ring 179, CH- 4054 Basel (CH)**

**EP 0 175 641 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein Verfahren zur Herstellung von Dimethylmaleinsäureanhydrid aus Maleinsäure, Fumarsäure und/oder Maleinsäureanhydrid bei erhöhten Temperaturen in Gegenwart katalytischer Mengen eines heterocyclischen Amidins oder Amidinsalzes.

Aus der DE-A-2 233 862 und DE-A-2 233 889 ist bekannt, dass durch die Umsetzung von 2 Mol Maleinsäure, Fumarsäure und/oder Maleinsäureanhydrid mit 1 Mol eines heterocyclischen Amidins oder Amidinsalzes bei erhöhten Temperaturen und anschliessende saure Hydrolyse Dimethylmaleinsäureanhydrid gebildet wird. Es wurde nun gefunden, dass die Verwendung katalytischer Mengen eines Amidins oder Amidinsalzes bei dieser Reaktion genügt, um in hohen Ausbeuten das gewünschte Dimethylmaleinsäureanhydrid zu erhalten und hierbei keine saure Hydrolyse erforderlich ist.

Gegenstand vorliegender Erfindung ist ein Verfahren zur Herstellung von Dimethylmaleinsäureanhydrid durch Umsetzung von 2 Äquivalenten Maleinsäure, Fumarsäure und/oder Maleinsäureanhydrid bei einer Temperatur von mindestens 90° C in Gegenwart eines Amidines und/oder Amidinsalzes der Formeln I und II

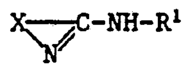 (I)

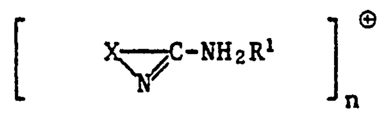 $Y^{n\ominus}$ (II),

worin $R^1$ ein Wasserstoffatom, Alkyl, Cycloalkyl, Carboxyalkyl, Aryl, Aralkyl, Alkaryl oder Alkaralkyl bedeutet, Y das Anion einer anorganischen, oder organischen Protonensäure ist, n eine ganze Zahl von 1-3 bedeutet, und X zusammen mit der Gruppierung

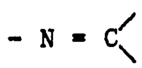

den Rest eines substituierten oder unsubstituierten 5- oder 6-gliedrigen Heteroringes bildet, der weitere Heteroatome enthalten kann, das dadurch gekennzeichnet ist, dass die Verbindungen der Formel I und II in katalytischen Mengen von 0,1 bis 15 Mol %, bezogen auf Maleinsäure, Fumarsäure und/oder Maleinsäureanhydrid, eingesetzt werden.

Vorzugweise verwendet man im erfindungsgemässen Verfahren Maleinsäure, Maleinsäureanhydrid oder 1 : 1-Gemische hiervon (Molverhältnis).

Als durch X zusammen mit der Gruppierung

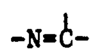

gebildete Reste eines 5- oder 6-gliedrigen, gegebenenfalls weitersubstituierten Heteroringes, der weitere Heteroatome enthalten kann, sind beispielsweise Imidazolyl-, Pyrazolyl-, Triazolyl-, Thiazolyl-, Isothiazolyl-, Oxadiazolyl-, Thiadiazolyl-, Oxazolyl-, Pyridinyl-, Pyridazinyl-, Pyrimidinyl-, Pyrazinyl- und Triazinylreste zu nennen.

Sind diese Reste weitersubstituiert, so können sie beispielsweise Halogene, wie Fluor, Chlor oder Brom, Phenylgruppen, Alkyl- oder Alkoxygruppen mit 1-4 Kohlenstoffatomen, Aminogruppen, Mono- oder Dialkylaminogruppen mit je 1-4 Kohlenstoffatomen im Alkylrest oder Hydroxylgruppen enthalten, oder aber mit weiteren homo- oder heterocyclischen Ringen kondensiert sein. Bevorzugte Substituenten sind Halogen, $C_1$-$C_4$-Alkyl und $C_1$-$C_4$-Alkoxy. Als Beispiele kondensierter 5- oder 6-gliedriger Heteroringsysteme seien genannt:

Benzimidazol, Benzthiazol, Benzoxazol, Pterin, Purin, Chinolin, Isochinolin, Naphthyridin, Phthalazin, Cinnolin, Chinazolin und Chinoxalin.

Vorzugsweise sind durch X zusammen mit der Gruppierung

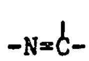

dargestellte Reste eines 5- oder 6-gliedrigen Heteroringes nicht weitersubstituiert. Bevorzugt ist der Heteroring ein Thiazolyl-2-Rest, besonders der Pyridinyl-2-Rest.

Die Amidine der Formel I sind bekannt oder können auf an sich bekannte Weise hergestellt werden. Als Beispiele geeigneter Verbindungen der Formel I seien genannt: 2-Aminoimidazol, 2-Aminobenzimidazol, 3-Aminopyrazol, 3-Amino-5-methylpyrazol, 3-Amino-4-brom-5-methylpyrazol, 3-Amino-1-phenylpyrazol, 3-Amino-1,2,4-triazol, 3,5-Diamino-1,2,4-triazol, 4-Amino-1,2,3-triazol, 2-Amino-1,3-thiazol, 3-Aminoisothiazol, 2-Amino-5-chlorthiazol, 2-Amino-4-phenylthiazol thiazol, 2-Amino-benzthiazol, 2-Amino-6-brombenzthiazol, 2-Amino-4,6-dibrombenzthiazol, 3-Amino-4-phenylfurazan, 3-Amino-4-methylfurazan, 3-Aminoisoxazol, 2-Aminooxazol, 2-Aminobenzoxazol, 2-Amino-pyridin, 2-Amino-3-methyl-, -4-methyl-oder -6-methylpyridin, 2-Amino-5-brompyridin, 2-Amino-5-brompyridin, 2-Amino-5-chlorpyridin, 2-Amino-3,5-dibrompyridin, 2-Amino-3,5-dichlorpyridin, 2-Amino-3-methylaminopyridin, 2,6-Diaminopyridin, 2,3-Diaminopyridin, 2-Aminopyrazin, 2-Aminopyrimidin, 6-Amino-2-chlorpyrimidin, 6-Amino-2,4-dimethylpyrimidin, 2-Amino-5-brom-4,6-dimethylpyrimidin, 2-Amino-6-chlorpyrimidin, 2-Amino-4,6-dichlorpyrimidin, 6-Amino-2,4-dichlorpyrimidin, 2-Amino-4,6-dimethylpyrimidin, 4,6-Diamino-pyrimidin, 6-Amino-4-methylpyrimidin, 3-Aminopyridazin, 2-Amino-1,3,5-triazin, 2,4,6-Triamino-1,3,5-triazin, 2-Amino-4,6-dichlor-1,3,5-triazin, 2-Amino-4,6-dimethyl-1,3,5-triazin, 4-Amino-6-hydroxy-2-methyl-1,3,5-triazin, 2,4-Diamino-6-methyl-1,3,5-triazin, 8-Aminopurin, 2-Aminpurin, 6-Aminopurin (Adenin), 2-Amino-6-brompurin, 2-Amino-6-chlorpurin, 6-Amino-2,8-dichlorpurin, 8-Amino-2,6-dichlorpurin, 6,Amino-2-methylpurin, 2,8-Diaminopurin, 6,8-Diaminopurin, 7-Methyl-2,6,8-triaminopurin, 1-Aminoisochinolin, 2-Aminochinolin, 2,4-Diaminochinolin, 2-Amino-1,7-naphthyridin, 2-Amino-1,5-naphthyridin, 2-Amino-6,7-dimethyl-1,8-naphthyridin, 2-Aminochinoxalin, 2,3-Diaminochinoxalin, 4-Aminochinazolin.

Werden im erfindungsgemässen Verfahren Amidinsalze der Formel II verwendet, so stellt n eine ganze Zahl von 1 bis 3 und Y vorzugsweise das Anion der Ameisen-, Essig-, Propion-, Chlorwasserstoff-, Bromwasserstoff-, Schwefel- oder Phosphorsäure dar. Ganz besonders bevorzugt ist Y jedoch das Anion einer Carbonsäure mit 2 bis 4 C-Atomen, besonders der Essigsäure (n = 1). Diese Salze können in üblicher Weise durch Behandeln des betreffenden Amidins der Formel I mit der entsprechenden Säure hergestellt werden. Diese Herstellung kann direkt in situ erfolgen, oder man kann das isolierte Salz für die Umsetzung verwenden.

$R^1$ als Alkyl kann linear oder verzweigt sein und enthält bevorzugt 1 bis 12, besonders 1 bis 6 C-Atome. Beispiele sind: Methyl, Ethyl, n-Propyl, i-Propyl, n-Butyl, i-Butyl, t-Butyl, Pentyl, Hexyl, Octyl, Decyl und Undecyl. $R^1$ als Cycloalkyl enthält bevorzugt 5 bis 7 Ringkohlenstoffatome. Beispiele sind Cyclopentyl und Cyclohexyl. $R^1$ als Carboxyalkyl enthält bevorzugt 2 bis 12, besonders 2 bis 6 C-Atome. Beispiele sind: Carboxymethyl, Carboxyethyl, Carboxypropyl, Carboxybutyl, Carboxypentyl, Carboxyheptyl, Carboxyundecyl.

$R^1$ als Aryl enthält bevorzugt 6 bis 12 C-Atome, als Aralkyl und Alkaryl 7 bis 16 C-Atome und als Alkaralkyl 8 bis 16 C-Atome. Die Reste können substituiert sein, z. B. durch Halogen, besonders Chlor, Carboxyl, $C_1$-$C_4$-Alkoxy, $NO_2$ oder -OH. Beispiele sind Phenyl, Benzyl, Methylphenyl, Methylbenzyl und Phenylethyl.

In einer bevorzugten Ausführungsform ist $R^1$ $C_1$-$C_4$-Alkyl oder unsubstituiertes oder durch ein Chloratom, eine Nitro- oder eine $C_1$-$C_4$-Alkoxygruppe, besonders eine Methoxygruppe, substituiertes Phenyl. Gemäss einer weiteren Bevorzugung stellt $R^1$ ein Wasserstoffatom dar.

Die Reaktionstemperatur beträgt bevorzugt 110 bis 160°C. Die Reaktion kann gegebenenfalls unter Druck durchgeführt werden. Die Verbindungen der Formeln I und II werden vorzugsweise in einer Menge von 1 bis 10 Mol % eingesetzt.

Die erfindungsgemässe Umsetzung kann in einem gegenüber den Reaktionspartnern inerten organischen Lösungsmittel, wie gegebenenfalls chlorierten aromatischen Kohlenwasserstoffen, z. B. Benzol, Toluol, Xylolen, Chlorbenzol oder Dichlorbenzolen, Dialkylsulfoxiden, z. B. Dimethylsulfoxid, Methylcellosolve, Hexamethylphosphorsäuretriamid, N,N-Dialkylamiden einer niederen Monocarbonsäure, z. B. Dimethylformamid oder Dimethylacetamid, oder niederen Dialkylestern der Kohlensäure, z. B. Dimethylcarbonat oder Diethylcarbonat, durchgeführt werden. Auch Gemische solcher Lösungsmittel können verwendet werden. Falls das Amidinsalz der allgemeinen Formel II direkt in situ hergestellt wird, kann gegebenenfalls auch die verwendete Säure, z. B. eine aliphatische $C_2$-$C_4$-Carbonsäure, besonders Essigsäure, als Lösungsmittel verwendet werden.

Gemäss einer bevorzugten Ausführungsform wird die erfindungsgemässe Umsetzung ohne Zusatz eines Lösungsmittels oder insbesondere in wasserfreier Essigsäure durchgeführt.

Dem Reaktionsgemisch kann auch eine Pufferverbindung, z. B. ein Alkaliacetat, wie Natriumacetat, zugesetzt werden. Bei alleiniger Verwendung von Maleinsäureanhydrid hat es sich als zweckmässig erwiesen, Wasser mitzuverwenden, vorteilhaft in einer Menge von 0,5 bis 20 Gew.-%, bezogen auf die Menge des eingesetzten Maleinsäureanhydrides.

Die Isolierung und Reinigung des Reaktionsproduktes erfolgen nach üblichen Methoden, z. B. Destillation, Wasserdampfdestillation, Extraktion oder Kristallisation. Es ist ein besonderer Vorteil des erfindungsgemässen Verfahrens, dass die Isolierung des Reaktionsproduktes direkt vorgenommen werden kann, ohne dass eine saure Hydrolyse durchgeführt werden muss und hierbei hohe Ausbeuten erzielt werden können. Die Amidinverbindungen können hierbei quantitativ zurückgewonnen werden.

Dimethylmaleinsäureanhydrid ist ein wertvolles Zwischenprodukt zur Herstellung von lichtempfindlichen Polymeren mit Dimethylmaleinimidylgruppen (vgl. DE-A-2 626 769).

Die nachfolgenden Beispiele erläutern die Erfindung näher.

**Beispiel 1**: 58,0 g (0,5 Mol) Maleinsäure und 0,85 g (5 mMol) 2-Phenyl-aminopyridin werden in 200 ml Eisessig 48 Stunden am Rückfluss gekocht. Anschliessend wird der Eisessig abdestilliert und der Rückstand wasserdampfdestilliert. Aus dem Destillat werden nach Filtration und Trocknen 13,2 g (42 %) Dimethylmaleinsäureanhydrid vom Smp. 91-93°C erhalten. Durch Extraktion der Mutterlauge mit Ether werden weitere 3,2 g (10 %) Dimethylmaleinsäureanhydrid isoliert.

**Beispiele 2-11**: Die Beispiele 2-11 werden unter den Reaktionsbedingungen gemäss Beispiel 1 unter Verwendung der in der Tabelle angegebenen Katalysatoren durchgeführt.

**Beispiel 2-11**

| Beispiel | Katalysator | Mol% | Ausbeute |
|---|---|---|---|
| 2 | 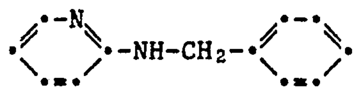 | 10 | 35,6 % |
| 3 | 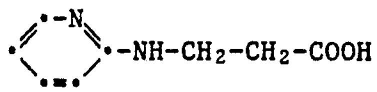 | 10 | 35,6 % |
| 4 | 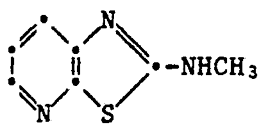 | 10 | 33,3 % |
| 5 | 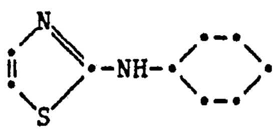 | 10 | 49,5 % |
| 6 | 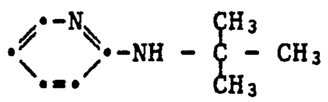 | 10 | 68,6 % |
| 7 | 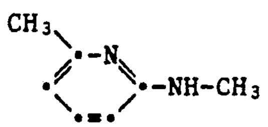 | 10 | 55,9 % |
| 8 | 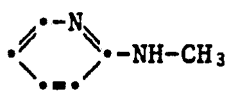 | 10 | 54,1 % |
| 9 | 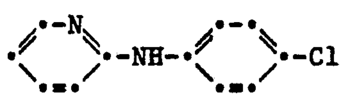 | 10 | 57,8 % |
| 10 | 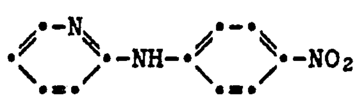 | 1 | 53 % |
| 11 | 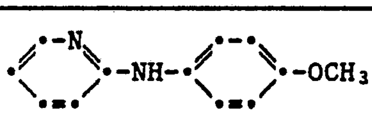 | 10 | 57,8 % |

**Beispiel 12**: Zu einer kochenden Lösung von 0,85 g (5 mMol) 2-Phenyl-aminopyridin in 200 ml Eisessig werden während 7 Stunden portionenweise 58,0 g (0,5 Mol) Maleinsäure gegeben. Anschliessend wird 14 Stunden am Rückfluss gekocht. Nach Aufarbeiten wie in Beispiel 1 angegeben, werden 11,2 g (35,6 %) Dimethylmaleinsäureanhydrid vom Smp. 91-93° C erhalten.

**Beispiel 13**: Zu einer kochenden Lösung von 1,7 g (0,01 Mol) 2-Phenyl-aminopyridin in 50 ml Eisessig werden gleichzeitig aus zwei Tropftrichtern innert einer Stunde 98,0 g (1 Mol) Maleinsäureanhydrid gelöst in 250 ml Eisessig und 9 ml Wasser getropft. Anschliessend wird 20 Stunden am Rückfluss gekocht. Nach Aufarbeiten wie in Beispiel 1 angegeben, werden 28,9 g (45,9 %) Dimethylmaleinsäureanhydrid vom Smp. 91-93° C erhalten.

**Beispiel 14**: Zu einer kochenden Lösung von 1,7 g (0,01 Mol) 2-Phenyl-aminopyridin in 50 ml Eisessig wird innert 3 Stunden eine Lösung von 49,0 g (0,5 Mol) Maleinsäureanhydrid und 58,0 g (0,5 Mol) Maleinsäure in 350 ml Eisessig getropft. Anschliessend wird 3 Stunden am Rückfluss gekocht. Nach Aufarbeiten wie in Beispiel 1 angegeben, werden 34,3 g (54,0 %) Dimethylmaleinsäureanhydrid vom Smp. 91-93° C erhalten.

**Beispiel 15**: Zu einer kochenden Mischung aus 1,7 g (0,01 Mol) 2-Phenyl-aminopyridin und 22,8 g Natriumacetat-Trihydrat in 100 ml Eisessig wird innert 1 Stunde eine Lösung von 98,0 g (1 Mol) Maleinsäureanhydrid in 200 ml Eisessig getropft. Anschliessend wird 18 Stunden am Rückfluss gekocht. Nach Aufarbeitung wie in Beispiel 1 beschrieben, werden 27,9 g (44,3 %) Dimethylmaleinsäureanhydrid vom Smp. 91-93°C erhalten.

**Beispiel 16**: 58,0 g (0,5 Mol) Maleinsäure und 4,7 g (0,05 Mol) 2-Aminopyridin werden in 200 ml Eisessig 48 Stunden am Rückfluss gekocht. Nach Aufarbeiten wie in Beispiel 1 angegeben, werden 17,0 g (54 %) Dimethylmaleinsäureanhydrid vom Smp. 91-93°C erhalten.

**Patentansprüche**

1. Verfahren zur Herstellung von Dimethylmaleinsäureanhydrid durch Umsetzung von 2 Äquivalenten Maleinsäure, Fumarsäure und/oder Maleinsäureanhydrid bei einer Temperatur von mindestens 90°C in Gegenwart eines Amidines und/oder Amidinsalzes der Formeln I und II

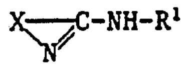 (I)

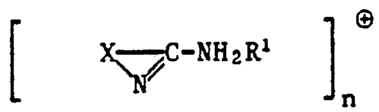 $Y^{n\ominus}$ (II),

worin $R^1$ ein Wasserstoffatom, Alkyl, Cycloalkyl, Carboxyalkyl, Aryl, Aralkyl, Alkaryl oder Alkaralkyl bedeutet, Y das Anion einer anorganischen oder organischen Protonensäure ist, n eine ganze Zahl von 1-3 bedeutet, und X zusammen mit der Gruppierung

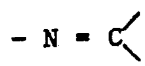

den Rest eines substituierten oder unsubstituierten 5- oder 6-gliedrigen Heterorings bildet, der weitere Heteroatome enthalten kann, dadurch gekennzeichnet, dass die Verbindungen der Formel I und II in katalytischen Mengen von 0,1 bis 15 Mol %, bezogen auf Maleinsäure, Fumarsäure und/oder Maleinsäureanhydrid, eingesetzt werden.

2. Verfahren gemäss Anspruch 1, dadurch gekennzeichnet, dass die Verbindungen der Formeln I und II in einer Menge von 1-10 Mol % eingesetzt werden.

3. Verfahren gemäss Anspruch 1, dadurch gekennzeichnet, dass es in Gegenwart eines inerten Lösungsmittels durchgeführt wird.

4. Verfahren gemäss Anspruch 3, dadurch gekennzeichnet, dass das Lösungsmittel eine aliphatische Carbonsäure mit 2 bis 4 C-Atomen, besonders Essigsäure, ist.

5. Verfahren gemäss Anspruch 1, dadurch gekennzeichnet, dass es bei Temperaturen von 110-160°C durchgeführt wird.

6. Verfahren gemäss Anspruch 1, dadurch gekennzeichnet, dass man bei alleiniger Verwendung von Maleinsäureanhydrid 0,5 bis 20 Gew.-% Wasser einsetzt, bezogen auf die Menge Maleinsäureanhydrid.

7. Verfahren gemäss Anspruch 1, dadurch gekennzeichnet, dass Maleinsäure, Maleinsäureanhydrid oder 1 : 1-Gemische (Molverhältnis) hiervon eingesetzt werden.

8. Verfahren gemäss Anspruch 1, dadurch gekennzeichnet, dass der Heteroring ein Pyridinyl-2-Rest oder ein Thiazolyl-2-Rest ist.

9. Verfahren gemäss Anspruch 8, dadurch gekennzeichnet, dass $R^1$ als von Wasserstoff verschiedener Rest $C_1$-$C_{12}$-Alkyl, $C_5$-$C_7$-Cycloalkyl, $C_2$ bis $C_{12}$-Carboxyalkyl, $C_6$-$C_{12}$-Aryl, $C_7$-$C_{16}$-Aralkyl, $C_7$-$C_{16}$-Alkaryl oder $C_8$-$C_{16}$-Alkaralkyl ist.

10. Verfahren gemäss Anspruch 8, dadurch gekennzeichnet, dass $R^1$ ein Wasserstoffatom ist.

**Claims**

1. A process for the preparation of dimethylmaleic anhydride by reacting 2 equivalents of maleic acid, fumaric acid and/or maleic anhydride at a temperature of at least 90°C and in the presence of an amidine and/or an amidine salt of the formulae I and II

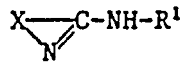

(I)

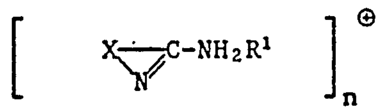

$Y^{n\ominus}$ (II),

wherein $R^1$ is a hydrogen atom, alkyl, cycloalkyl, carboxyalkyl, aryl, aralkyl, alkaryl or alkaralkyl, Y is the anion of an inorganic or organic protonic acid, n is an integer from 1 to 3, and X, together with the group

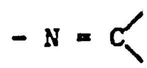

forms the radical of a substituted or unsubstituted 5- or 6-membered heterocyclic ring whith may contain further hetero atoms, which process comprises employing the compounds of formulas I and II in catalytic amounts of 0.1 to 15 mol %, based on maleic acid, fumaric acid and/or maleic anhydride.

2. A process according to Claim 1, wherein the compounds of formulas 1 and II are employed in an amount of 1 to 10 mol %.

3. A process according to Claim 1, which is carried out in the presence of an inert solvent.

4. A process according to Claim 3, wherein the solvent is an aliphatic carboxylic acid having 2 to 4 carbon atoms, in particular acetic acid.

5. A process according to Claim 1, which is carried out at temperatures from 110 to 160° C.

6. A process according to Claim 1, wherein 0.5 to 20 % by weight of water, based on the amount of maleic anhydride, is added if said maleic anhydride is employed alone.

7. A process according to Claim 1, which comprises the use of maleic acid, maleic anhydride or a 1 : 1 mixture (molar ratio) thereof.

8. A process according to Claim 1, wherein the heterocyclic ring is a 2-pyridinyl radical or a 2-thiazolyl radical.

9. A process according to Claim 8, wherein $R^1$ as a radical other than hydrogen is $C_1$-$C_{12}$alkyl, $C_5$-$C_7$cycloalkyl, $C_2$-$C_{12}$carboxyalkyl, $C_6$-$C_{12}$aryl, $C_7$-$C_{16}$aralkyl, $C_7$-$C_{16}$alkaryl or $C_8$-$C_{16}$alkaralkyl.

10. A process according to Claim 8, wherein $R^1$ is a hydrogen atom.

**Revendications**

1. Procédé pour préparer l'anhydride diméthylmaléique par réaction de deux équivalents d'acide maléique, d'acide fumarique et/ou d'anhydride maléique, à une température d'au moins 90° C, en présence d'une amidine et/ou d'un sel d'amidine répondant respectivement aux formules I et II:

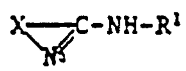

(I)

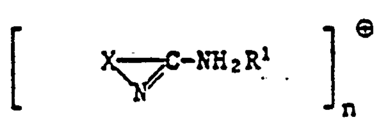

$Y^{n\ominus}$ (II),

dans lesquelles:

$R^1$ représente un atome d'hydrogène ou un radical alkyle, cycloalkyle, carboxyalkyle, aryle, aralkyle, alkylaryle ou alkyl-aralkyle,

Y représente l'anion d'un acide protonique minéral ou organique,

n représente un nombre entier de 1 à 3 et

X forme, avec le groupement:

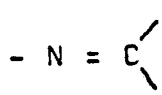

le radical d'un hétérocycle à 5 ou 6 maillons, substitué ou non, qui peut contenir d'autres hétéroatomes, procédé caractérisé en ce que les composés de formules I et II sont mis en jeu en des quantités catalytiques, en l'espèce en des quantités de 0,1 à 15 % en moles par rapport à l'acide maléique, à l'acide fumarique et/ou à

l'anhydride maléique.

2. Procédé selon le revendication 1 caractérisé en ce que les composés de formules I et II sont mis en jeu en une quantité de 1 à 10 % en moles.

3. Procédé selon la revendication 1 caractérisé en ce qu'on l'exécute en présence d'un solvant inerte.

4. Procédé selon la revendication 3 caractérisé en ce que le solvant est un acide carboxylique aliphatique contenant de 2 à 4 atomes de carbone, plus particulièrement l'acide acétique.

5. Procédé selon la revendication 1 caractérisé en ce qu'on l'exécute à des températures de 110 à 160° C.

6. Procédé selon la revendication 1 caractérisé en ce qu'on utilise seulement l'anhydride maléique et en ce qu'on utilise de 0,5 à 20 % en poids d'eau par rapport à la quantité de l'anhydride maléique.

7. Procédé selon la revendication 1 caractérisé en ce qu'on utilise l'acide maléique, l'anhydride maléique ou un mélange de ceux-ci au rapport molaire 1 : 1.

8. Procédé selon la revendication 1 caractérisé en ce que l'hétérocycle est un radical pyridyle-2 ou un radical thiazolyle-2.

9. Procédé selon la revendication 8 caractérisé en ce que $R^1$, en tant que radical autre que l'hydrogène, est un alkyle en $C_1$ - $C_{12}$, un cycloalkyle en $C_5$-$C_7$, un carboxyalkyle en $C_2$-$C_{12}$, un aryle en $C_6$-$C_{12}$, un aralkyle en $C_7$-$C_{16}$, un alkylaryle en $C_7$-$C_{16}$ ou un alkyl-aralkyle en $C_8$-$C_{16}$.

10. Procédé selon la revendication 8 caractérisé en ce que $R^1$ est un atome d'hydrogène.